# EUROPEAN PATENT APPLICATION

(11) **EP 4 403 167 A1**
(43) Date of publication of application: **24.07.2024**
(21) Application number: 21957132.0
(22) Date of filing: 17.09.2021
(51) Int. Cl.: A61K 9/22, C07D 215/36, C07D 215/06, C07D 215/42, A61K 31/435, A61K 31/4353, A61K 31/4375, A61K 47/10, A61K 47/12, A61K 47/14, A61K 47/26, A61K 47/36, A61K 47/38, A61P 19/06

(54) **QUINOLINE COMPOUND SUSTAINED-RELEASE TABLET AND PREPARATION METHOD THEREFOR**

(71) Applicant: Hinova Pharmaceuticals Inc., Chengdu, Sichuan 610041 (CN)
(72) Inventor: WEI, Xing, Chengdu, Sichuan 610041 (CN); KUANG, Tongtao, Chengdu, Sichuan 610041 (CN); CHEN, Jiang, Chengdu, Sichuan 610041 (CN); AI, Chaowu, Chengdu, Sichuan 610041 (CN); LI, Xinghai, Chengdu, Sichuan 610041 (CN)
(74) Representative: Altmann Stößel Dick Patentanwälte PartG mbB
(86) International application number: PCT/CN2021/119132
(87) International publication number: WO 2023/039850

(57) **Abstract**

Disclosed in the present invention is a quinoline compound sustained-release tablet, which contains the following raw and auxiliary materials in parts by weight: 1-40 parts of a quinoline compound, 100-300 parts of a filler, 50-200 parts of a sustained-release material and 0.5-4 parts of a lubricant. The filler comprises, but is not limited to, mannitol, microcrystalline cellulose, lactose, starch, corn starch, calcium hydrogen phosphate hydrate, magnesium carbonate, calcium carbonate, purified sucrose and/or glucose; the sustained-release material comprises, but is not limited to, hydroxypropyl methylcellulose, hydroxypropyl cellulose, and povidone, glyceryl behenate, and/or a long-chain fatty acid; and the lubricant comprises, but is not limited to, magnesium stearate, calcium stearate, sucrose fatty acid ester, sodium stearyl fumarate, polyethylene glycol, talc, glyceryl behenate and/or stearic acid.

## Description

### Technology of the invention

The present invention specifically relates to a quinoline compound sustained-release tablet and a preparation method therefor, and belongs to the pharmaceutical field.

### Background of the invention

Quinoline compounds are the inhibitors of urate anion transporter 1 (URAT1), which are self-developed by Hinova Pharmaceuticals Inc., possessing independent intellectual property rights (currently, 8 invention patents have been applied), and used for the treatment of hyperuricemia and gout. The active ingredients of quinoline compound sustained-release tablets are quinoline compounds, having a structure represented by formula I:

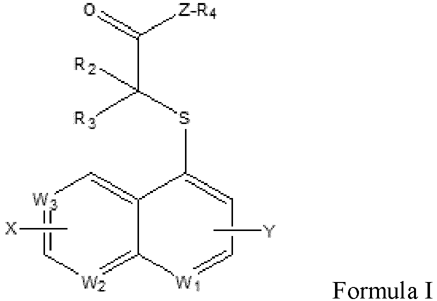

Application number: 201611109936.3, invention name: Quinoline compounds, preparation method thereof, and use thereof as urate transporter inhibitor drug.

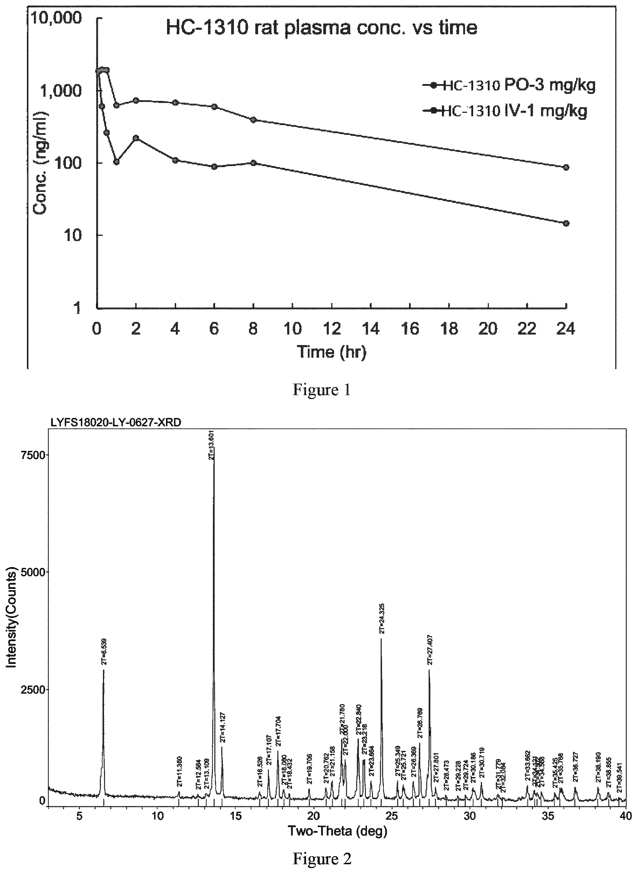

According to the PK test results of the drug in rats (Figure 1), the drug is quickly absorbed after entering the rat body, and the rats have a high plasma concentration for 4 hours after receiving the drug. Based on the pharmacological experiments, it is concluded that the excessive plasma concentration can cause potential toxic side effects. The CACO-2 experiment has shown that quinoline compounds exhibit high permeability in CACO-2 cells and are not substrates for efflux transporters. The solubility test has indicated that quinoline compounds have low solubility in water, and so they are classified as BCS class 2 according to biopharmaceutics classification system. Drug release is the rate-limiting process for drug absorption. Generally, there is a high correlation between the *in vitro* drug release testing and the *in vivo* drug bioavailability. Therefore, the development of sustained-release formulations can effectively reduce the plasma concentration after medication and the potential toxic side effects caused by Cₘₐₓ in the human body, while ensure the bioavailability.

### Content of the invention

The object of the present invention is to provide a quinoline compound sustained-release tablet and a preparation method therefor. In the present invention, the concept of sustained-release formulations is used in the development of the compound preparations, with the development goal of administrating once a day, effectively reducing Cₘₐₓ while maintaining AUC unchanged. The determined formulation is fully released within 12-20 h, improves drug compliance, reduces the incidence of adverse reactions, and increases drug stability.

The present invention provides a quinoline compound sustained-release tablet, which contains the following raw and auxiliary materials in parts by weight:
1-40 parts of quinoline compounds, 100-300 parts of fillers, 50-200 parts of sustained-release materials and 0.5-4 parts of lubricants;
the filler comprises, but is not limited to, mannitol, microcrystalline cellulose, lactose, starch, corn starch, calcium hydrogen phosphate hydrate, magnesium carbonate, calcium carbonate, purified sucrose and/or glucose;
the sustained-release material comprises, but is not limited to, hydroxypropyl methylcellulose, hydroxypropyl cellulose, povidones, glyceryl behenate, and/or long-chain fatty acids;
the lubricant comprises, but is not limited to, magnesium stearate, calcium stearate, sucrose fatty acid ester, sodium stearyl fumarate, polyethylene glycol, talc, glyceryl behenate and/or stearic acid.

Further, the filler is lactose and/or microcrystalline cellulose, and preferably, the filler is composed of lactose and microcrystalline cellulose at a mass ratio of 10-30:2-10; the sustained-release material is hydroxypropyl methylcellulose; the lubricant includes but is not limited to magnesium stearate, sodium fumarate, glyceryl behenate, and/or stearic acid.

More further, said lactose includes but is not limited to lactose monohydrate, anhydrous lactose, and/or lactose with different particle sizes; said microcrystalline cellulose includes but is not limited to microcrystalline cellulose PH101, PH102, PH301, and/or PH302; said hydroxypropyl methylcellulose includes but is not limited to hydroxypropyl methylcellulose K4M, K100LV, and/or K750; and said lubricant is magnesium stearate.

More further, the sustained-release tablet comprises the following raw and auxiliary materials in parts by weight:
5 parts of quinoline compounds, 256 parts of fillers, 117 parts of sustained-release materials and 1.9 parts of lubricants;
the filler is composed of lactose monohydrate and microcrystalline cellulose PH101 at a mass ratio of 10-70:2-30;
said hydroxypropyl methylcellulose is composed of hydroxypropyl methylcellulose K4M and K100LV, and their mass ratio is preferably 57:60;
and said lubricant is magnesium stearate.

Further, the quinoline compound is a compound as represented by formula I, or an optical isomer thereof, or a solvate thereof, or a pharmaceutically acceptable salt thereof, or a prodrug thereof; 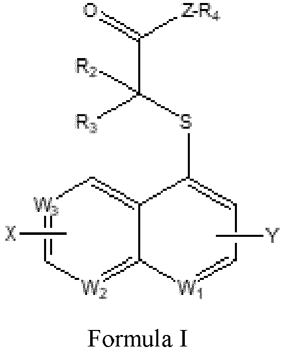 wherein:
Z is selected from O, S or -NH-;
W₁ is selected from N or CR^{a}; W₂ is selected from N or CR^{b}; W₃ is selected from N or CR^{c};
R^{a}, R^{b}, R^{c}, R₂, and R₃ are each independently selected from the group consisting of hydrogen, halogen, cyano, nitro, alkyl, alkenyl, alkynyl, cycloalkyl, heterocyclyl, aryl, heteroaryl, -OR^{d}, -S(O)ₘR^{d}, -C(O)R^{d}, C(O)OR^{d}, -C(O)NR^{e}R^{f}, -NR^{e}R^{f} or NR^{e}C(O)R^{f}, in which said alkyl, alkenyl, alkynyl, cycloalkyl, heterocyclyl, aryl or heteroaryl are each independently and optionally further substituted with one or more of the substituents selected from the group consisting of halogen, cyano, nitro, oxo, alkyl, haloalkyl, hydroxyalkyl, alkenyl, alkynyl, cycloalkyl, heterocyclyl, aryl, heteroaryl, -OR^{d}, -S(O)ₘR^{d}, -C(O)R^{d}, C(O)OR^{d}, -C(O)NR^{e}R^{f}, -NR^{e}R^{f} or NR^{e}C(O)R^{f};
R^{d} is selected from the group consisting of hydrogen, halogen, alkyl, cycloalkyl, heterocyclyl, aryl or heteroaryl, wherein said alkyl, cycloalkyl, heterocyclyl, aryl or heteroaryl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, carboxylic ester group, -C(O)NR^{e}R^{f}, -NR^{e}R^{f} or NR^{e}C(O)R^{f};
R^{e} and R^{f} are each independently selected from the group consisting of hydrogen, alkyl, cycloalkyl, heterocyclyl, aryl or heteroaryl, wherein said alkyl, cycloalkyl, heterocyclyl, aryl or heteroaryl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, and carboxylic ester group; and m is 0, 1, or 2;
X and Y are each independently selected from the group consisting of hydrogen, halogen, cyano, nitro, alkyl, cycloalkyl, haloalkyl and hydroxyalkyl;
Provided that Z is selected from O or S, R₄ is selected from hydrogen or C₁-C₆ alkyl, cycloalkyl, wherein said alkyl and cycloalkyl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, and carboxylic ester group, -C(O)NR^{e}R^{f}, -NR^{e}R^{f} or NR^{e}C(O)R^{f}; provided that Z is -NH-, R₄ is selected from the group consisting of hydrogen, aryl or heteroaryl, and preferably pyridinyl.

More further, the quinoline compound is 2-[4-(6-bromoquinolyl)-thio]-2-ethylbutyric acid and/or an optical isomer, a solvate, a pharmaceutically acceptable salt, and a prodrug thereof; the structure of said 2-[4-(6-bromoquinolyl)-thio]-2-ethylbutyric acid is as follows:

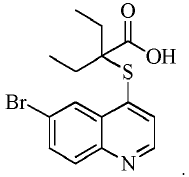

More further, the quinoline compound is crystalline form I, crystalline form II and/or a sodium salt crystalline form of 2-[4-(6-bromoquinolyl)-thio]-2-ethylbutyric acid.

More further, there are characteristic peaks where diffraction angles 2θ are 6.5±0.2°, 13.6±0.2°, 14.1±0.2°, 17.7±0.2°, 21.8±0.2°, 22.0±0.2°, 22.8±0.2°, 23.2±0.2°, 24.3±0.2°, 26.8±0.2°, and 27.4±0.2° in the X-ray powder diffraction pattern of the crystalline form I of 2-[4-(6-bromoquinolyl)thio]-2-ethylbutyric acid.

More further, the relative intensity of the characteristic peak is:

| Diffraction angle 2θ | Relative intensity% |
|---|---|
| 6.5±0.2 | 36.1 |
| 13.6±0.2 | 100.0 |
| 14.1±0.2 | 14.1 |
| 17.7±0.2 | 13.6 |
| 21.8±0.2 | 17.0 |
| 22.0±0.2 | 11.1 |
| 22.8±0.2 | 16.9 |
| 23.2±0.2 | 11.1 |
| 24.3±0.2 | 45.6 |
| 26.8±0.2 | 15.7 |
| 27.4±0.2 | 36.5. |

More further, there are characteristic peaks where diffraction angles 2θ are 7.9±0.2°, 9.5±0.2°, 15.9±0.2°, 18.2±0.2°, 19.1±0.2°, 23.9±0.2°, and 26.1±0.2° in the X-ray powder diffraction pattern of the crystalline form II of 2-[4-(6-bromoquinolyl)thio]-2-ethylbutyric acid.

More further, the relative intensity of the characteristic peak is:

| Diffraction angle 2θ | Relative intensity% |
|---|---|
| 7.9±0.2100.0 | 10.0 |
| 9.5±0.27.3 | |
| 15.9±0.228.6 | |
| 18.2±0.2 | |
| 19.1±0.26.8 | |
| 23.9±0.222.7 | |
| 26.1±0.26.7. | |

More further, there are characteristic peaks where diffraction angles 2θ are 6.1±0.2°, 10.5±0.2°, 12.0±0.2°, 14.1±0.2°, 15.9±0.2°, 18.0±0.2°, 21.7±0.2°, 27.6±0.2°, 32.0±0.2°, 33.8±0.2°, and 36.4±0.2° in the X-ray powder diffraction pattern of the sodium salt crystalline form of 2-[4-(6-bromoquinolyl)thio]-2-ethylbutyric acid.

More further, the relative intensity of the characteristic peak is:

| Diffraction angle 2θ | Relative intensity% |
|---|---|
| 6.1±0.222.2 | |
| 10.5±0.221.4 | |
| 12.0±0.230.7 | |
| 14.1±0.224.3 | |
| 15.9±0.289.9 | |
| 18.0±0.238.5 | |
| 21.7±0.227.9 | |
| 27.6±0.2 | 100.0 |
| 32.0±0.2 | 22.0 |
| 33.8±0.2 | 34.2 |
| 36.4±0.2 | 22.4. |

The present invention also provides a method for preparation of the sustained-release tablet mentioned above, and the preparation of crystalline form I of 2-[4-(6-bromoquinolyl)-thio]- 2-ethylbutyric acid comprises the following steps:
the crude product of 2-[4-(6-bromoquinolyl)-thio]-2-ethylbutyric acid was dissolved in methanol, and then filtered. The filtrate was naturally cooled and allowed to crystallize for 20-24 h. Then, the temperature was controlled to be 15-20 °C, and the filtrate was stirred and allowed to crystallized for 2 h, followed by filtration. The crystals were dried to obtain crystalline form I.

The present invention also provides a method for preparation of the sustained-release tablet mentioned above, and the preparation of crystalline form II of 2-[4-(6-bromoquinolyl)-thio]- 2-ethylbutyric acid comprises the following steps:
the crude product of 2-[4-(6-bromoquinolyl)-thio]-2-ethylbutyric acid was dissolved in a mixed solution of tetrahydrofuran and dichloromethane (7:4, v/v), and then filtered. The filtrate was evaporated to dry at room temperature to obtain crystalline form II.

The present invention also provides a method for preparation of the sustained-release tablet mentioned above, and the preparation of said sodium salt crystalline form of 2-[4-(6-bromoquinolyl)-thio]-2-ethylbutyric acid comprises the following steps:
the crude product of 2-[4-(6-bromoquinolyl)-thio]-2-ethylbutyric acid was dissolved in water, and the pH value was adjusted to be 12 with NaOH aqueous solution. The resultant solution was stirred for 3 min and filtered. The crystal was dried *in vacuum* to obtain the sodium salt crystalline form.

The present invention also provides a method for preparation of the quinoline compound sustained-release tablet mentioned above, which is prepared using wet granulation, dry granulation, direct powder compression or membrane controlled-release techniques, and preferably wet granulation.

Further, the wet granulation comprises the following steps:
a) The raw and adjuvant materials are weighed according to the ratio in the formula, and lactose is passed through a 60-mesh sieve;
b) 1/4-1/3 of the pre-determined amount of lactose in the formula is placed in a wet granulator, stirred for 5-10 min, and then 1/4-1/3 of the pre-determined amount of lactose and 1/3-1/2 of the pre-determined amount of quinoline compound are added and stirred for 5-10 min. Finally, the remaining amounts of lactose and quinoline compounds are added, and stirred for 5-10 min. Then, microcrystalline cellulose and hydroxypropyl methylcellulose are added and stirred for 10-20 min;
c) Water is added to make soft materials and granules, and then wet granules are dried to a moisture content of 1.0%~4.0%, followed by sorting;
d) Magnesium stearate is mixed with the granules prepared in step c, and then the mixture is pressed and coated to obtain the tablets.

More further, in step b, 1/3 of the pre-determined amount of lactose in the formula is placed in a wet granulator, stirred for 5 min, and then 1/3 of the pre-determined amount of lactose and 1/2 of the pre-determined amount of quinoline compound are added and stirred for 5 min. Finally, the remaining amounts of lactose and quinoline compounds are added, and stirred for 5 min. Then, microcrystalline cellulose and hydroxypropyl methylcellulose are added and stirred for 10 min.

More further, in step c, the granulation process involves passing through a 20-mesh sieve, the drying is carried out at 60 °C, and the sorting is performed with a 20-mesh sieve; and/or, in step d, the tablet is coated at a weight gain of 2-4%.

The present invention also provides a crystalline form of 2-[4-(6-bromoquinolyl)-thio]-2-ethylbutyric acid, which is crystalline form I of 2-[4-(6-bromoquinolyl)-thio]-2-ethylbutyric acid, and there are characteristic peaks where diffraction angles 2θ are 6.5±0.2°, 13.6±0.2°, 14.1±0.2°, 17.7±0.2°, 21.8±0.2°, 22.0±0.2°, 22.8±0.2°, 23.2±0.2°, 24.3±0.2°, 26.8±0.2°, and 27.4±0.2° in the X-ray powder diffraction pattern thereof.

Further, the relative intensity of the characteristic peaks is:

| Diffraction angle 2θ | Relative intensity% |
|---|---|
| 6.5±0.2 | 36.1 |
| 13.6±0.2 | 100.0 |
| 14.1±0.2 | 14.1 |
| 17.7±0.2 | 13.6 |
| 21.8±0.2 | 17.0 |
| 22.0±0.2 | 11.1 |
| 22.8±0.2 | 16.9 |
| 23.2±0.2 | 11.1 |
| 24.3±0.2 | 45.6 |
| 26.8±0.2 | 15.7 |
| 27.4±0.2 | 36.5 |

The present invention also provides a crystalline form of 2-[4-(6-bromoquinolyl)-thio]-2-ethylbutyric acid, which is crystalline form II of 2-[4-(6-bromoquinolyl)-thio]-2-ethylbutyric acid, and there are characteristic peaks where diffraction angles 2θ are 7.9±0.2°, 9.5±0.2°, 15.9±0.2°, 18.2±0.2°, 19.1±0.2°, 23.9±0.2°, and 26.1±0.2° in the X-ray powder diffraction pattern thereof.

Further, the relative intensity of the characteristic peaks is:

| Diffraction angle 2θ | Relative intensity% |
|---|---|
| 7.9±0.2100.0 | |
| 9.5±0.27.3 | |
| 15.9±0.228.6 | |
| 18.2±0.2 | 10.0 |
| 19.1±0.26.8 | |
| 23.9±0.222.7 | |
| 26.1±0.26.7. | |

The present invention also provides a crystalline form of 2-[4-(6-bromoquinolyl)-thio]-2-ethylbutyric acid, which is the sodium salt crystalline form of 2-[4-(6-bromoquinolyl)-thio]-2-ethylbutyric acid, and there are characteristic peaks where diffraction angles are 6.1±0.2°, 10.5±0.2°, 12.0±0.2°, 14.1±0.2°, 15.9±0.2°, 18.0±0.2°, 21.7±0.2°, 27.6±0.2°, 32.0±0.2°, 33.8±0.2°, and 36.4±0.2° in the X-ray powder diffraction pattern thereof.

Further, the relative intensity of the characteristic peaks is:

| Diffraction angle 2θ | Relative intensity% |
|---|---|
| 6.1±0.222.2 | |
| 10.5±0.221.4 | |
| 12.0±0.230.7 | |
| 14.1±0.224.3 | |
| 15.9±0.289.9 | |
| 18.0±0.238.5 | |
| 21.7±0.227.9 | |
| 27.6±0.2 | 100.0 |
| 32.0±0.2 | 22.0 |
| 33.8±0.2 | 34.2 |
| 36.4±0.2 | 22.4. |

The present invention has the following advantages:
1. Under the conditions of ensuring that the drug bioavailability is not decreased, the highest plasma concentration is reduced, avoiding potential toxic side effects;
2. Administrating once a day greatly improves patient compliance;
3. The selected excipients are all within the safe dosage specified by FDA and SFDA, and have good compatibility with the active ingredients, resulting in high product stability;
4. The sustained-release tablets of quinoline compounds are prepared using common solid preparation techniques, which have high production efficiency and low costs.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The *in vivo* drug time curve of active ingredient (formerly code-named HC-1310) in rats
Figure 2. XRD pattern of crystalline form I.
Figure 3. DSC spectroscopy of crystalline form I.
Figure 4. TGA spectrum of crystalline form I.
Figure 5. PLM pattern of crystalline form I.
Figure 6. XRD pattern of crystalline form II.
Figure 7. DSC spectroscopy of crystalline form II.
Figure 8. TGA spectrum of crystalline form II.
Figure 9. PLM pattern of crystalline form II.
Figure 10. XRD pattern of sodium salt crystalline form.
Figure 11. DSC spectroscopy of sodium salt crystalline form.
Figure 12. TGA spectrum of sodium salt crystalline form.
Figure 13. Screening of sustained-release material types and release curves of samples at different ratios.
Figure 14. Comparison of release curves of quinoline compound sustained-release tablets (specification: 5 mg) coated and not coated (plain tablets).
Figure 15. The mean plasma concentration-time curve of quinoline compound suspension or sustained-release tablets in male and female beagle dogs after a single oral administration at 5 mg/kg.

### Examples

### Example 1. Preparation of crystalline form I of quinoline compounds

59.2 kg of anhydrous methanol was added to a tank reactor, and then stirred. The reaction system was heated to reflux, to which was added 1135 g of crude 2-[4-(6-bromoquinolyl)thio]-2-ethylbutyric acid, and then heated to 65-70 °C to dissolve; the resultant solution was filtered to remove insoluble substances, and then the filtrate was transferred to the tank reactor, followed by heating the reaction system to 65-70 °C to dissolve and allowing the solution to become clear. The heating was turned off, and the solution was naturally cooled for crystallization for 20-24 h. The temperature of the reaction system was controlled to be 15-20 °C, and the system was stirred for crystallizing for 2 h. The system was filtered and dried to obtain the product crystalline form I of compound 2-[4-(6-bromoquinolyl)-thio]-2-ethylbutyric acid as off-white to white crystalline granules;

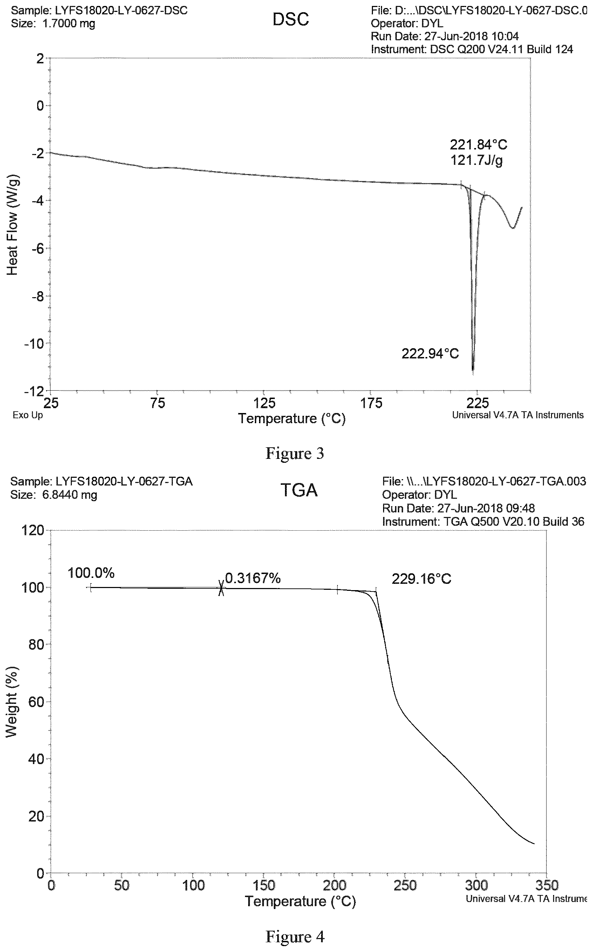

XRD, DSC, TGA, and PLM spectra of crystalline form I are shown in Figures 2 to 5.

### Example 2. Preparation of crystalline form II of quinoline compounds

About 10 mg of crude 2-[4-(6-bromoquinolyl)-thio]-2-ethylbutyric acid was added to 0.7 mL of tetrahydrofuran and 0.4 mL of dichloromethane, and then the solution was allowed to dissolve and become clear, followed by filtering. The filtrate was placed in a fume hood, and evaporated in an open state at room temperature to obtain the product of crystalline form II of 2-[4-(6-bromoquinolyl)-thio]-2-ethylbutyric acid;

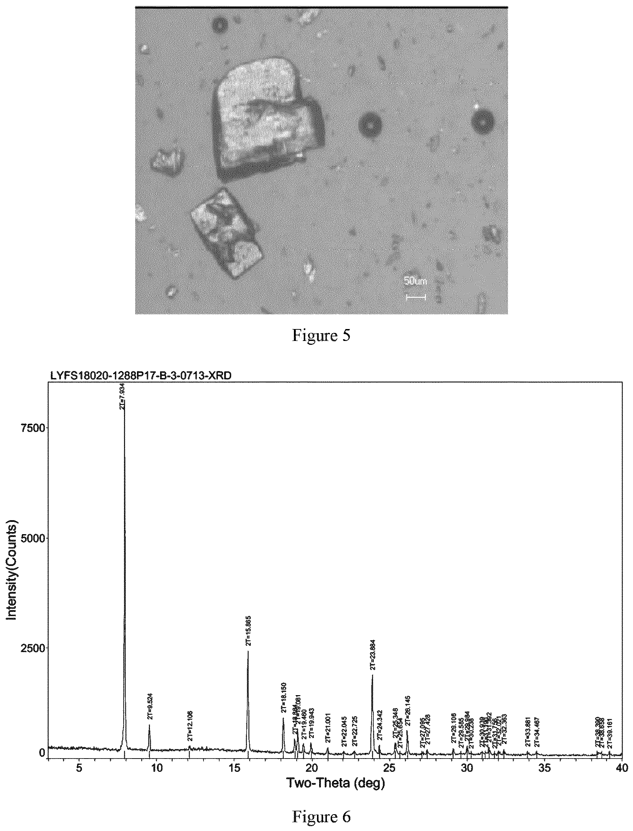

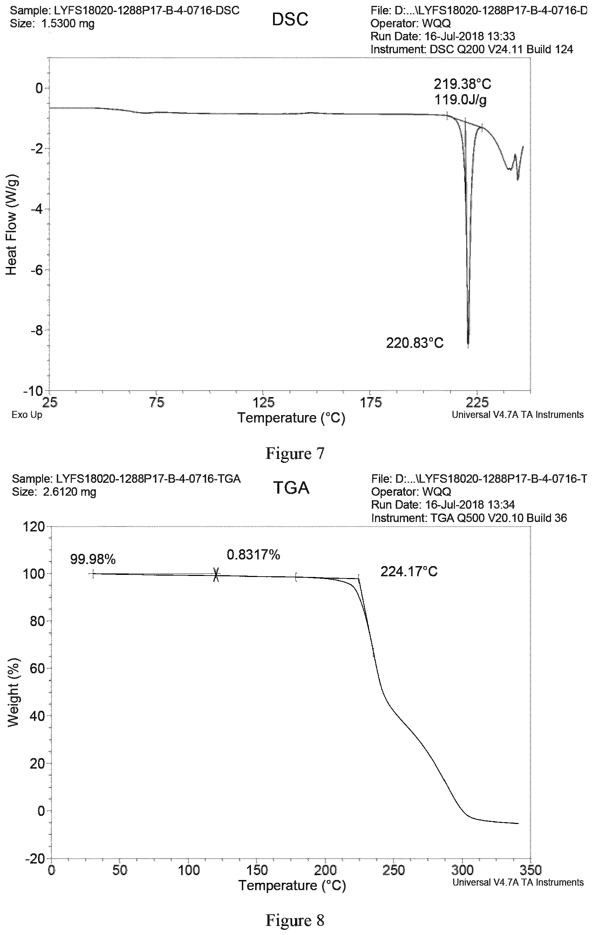

XRD, DSC, TGA, and PLM spectra of crystalline form II are shown in Figures 6 to 9.

### Example 3. Preparation of sodium salt crystalline form of quinoline compounds

About 300 mg of crude 2-[4-(6-bromoquinolyl)-thio]-2-ethylbutyric acid was added into 12 mL of water, and then stirred, to which was added 1 mol/L of NaOH aqueous solution dropwise to pH 12. The sample was allowed to precipitate under stirring. After stirring for additional 3 min, the solution was filtered and dried overnight *in vacuum,* to obtain the sodium salt crystalline form of compound 2-[4-(6-bromoquinolyl)-thio]-2-ethylbutyric acid;

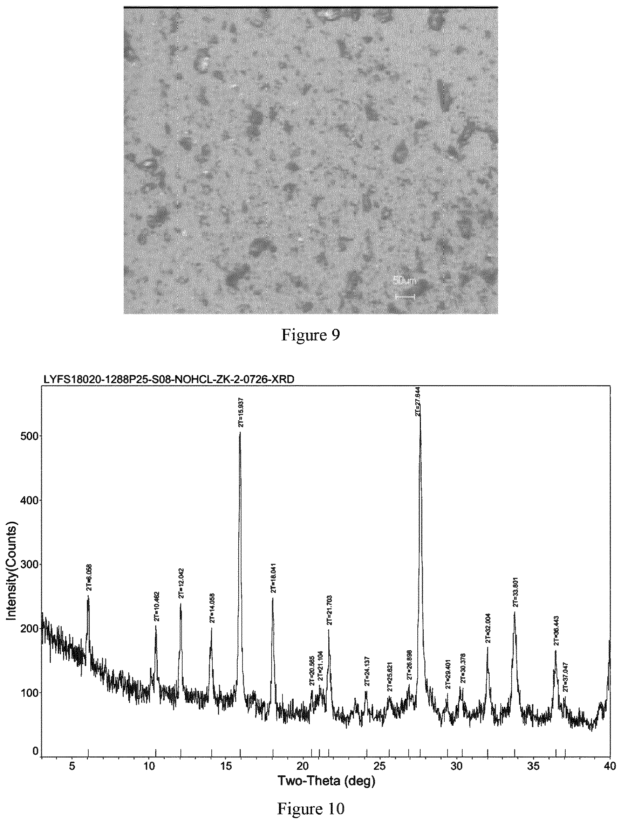

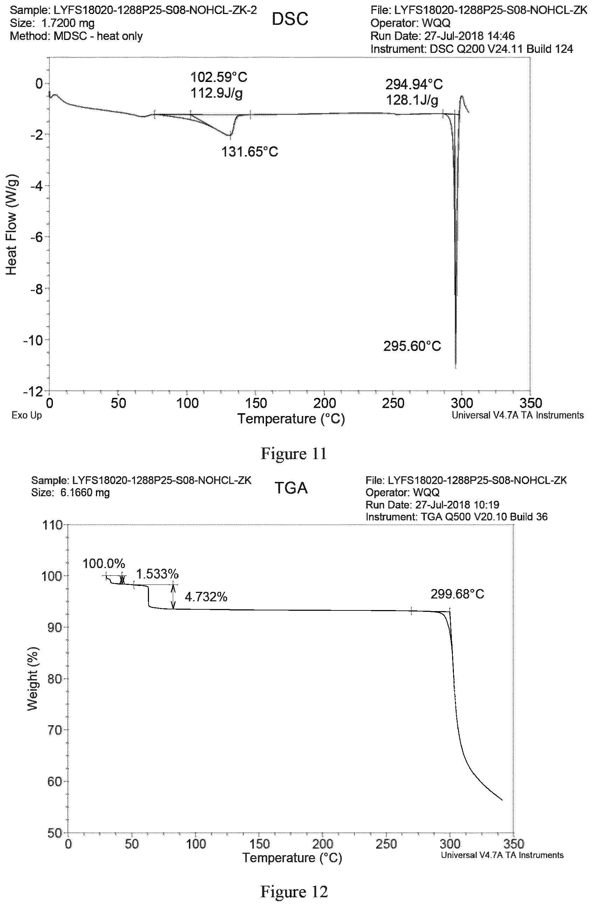

XRD, DSC, and TGA spectra of sodium salt crystalline form are shown in Figures 10 to 12.

### Example 4. Preparation of quinoline compound sustained-release tablets

Formula: 5 g of crystalline form I of compound 2-[4-(6-bromoquinolyl)-thio]-2-ethylbutyric acid prepared in Example 1, 226.1 g of lactose monohydrate, 30 g of microcrystalline cellulose PH101, 57 g of hydroxypropyl methylcellulose K4M (HPMC K4M), 60 g of hydroxypropyl methylcellulose K100LV (HPMC K100LV), and 1.9 g of magnesium stearate.

### Preparation method:

a. The raw and adjuvant materials were weighed according to the ratio, and lactose was passed through a 60-mesh sieve;
b. 1/3 of the pre-determined amount of lactose in the formula was placed in a wet granulator, stirred for 5 min, and then 1/3 of the pre-determined amount of lactose and 1/2 of the pre-determined amount of quinoline compound were added and stirred for 5 min. Finally, the remaining amounts of lactose and quinoline compounds were added, and stirred for 5 min. Then, microcrystalline cellulose and hydroxypropyl methylcellulose were added and stirred for 10 min;
c. Water was added to make soft materials, the granulation process was carried out by passing through a 20-mesh sieve, the materials were dried at 60 °C to 3% moisture content, and then the sorting was performed with a 20-mesh sieve;
d. Magnesium stearate was mixed with the granules obtained in step c, and then pressed into tablets. The tablets were coated at a weight gain of 3%, to obtain the sustained-release tablets.

The beneficial effects of the present invention were further demonstrated by reference to the following Experimental examples:

### Experiment example 1: Comparison of different ratios and types of sustained-release materials used for quinoline compound sustained-release tablets

### 1. Formula

See Table 1.

**Table 1. Release comparison of different ratios and types of sustained-release materials used in quinoline compound sustained-release tablets (specification: 5 mg).**

| Components (100 tablets) | 17030601 (mg/tablet) | 17030602 (mg/tablet) | 17030603 (mg/tablet) | 17030604 (mg/tablet) | 17030605 (mg/tablet) |
|---|---|---|---|---|---|
| API | 5 | 5 | 5 | 5 | 5 |
| HPMC K750 | 120 | 160 | - | - | - |
| HPMC K4M | - | - | 120 | 160 | - |
| HPC HXF (Hydroxypropyl cellulose HXF) | - | - | - | - | 120 |
| Lactose monohydrate | 255 | 215 | 255 | 215 | 255 |
| 10% PVPK30 aqueous solution | 141.6 | 139.8 | 142.8 | 140.3 | 49.6 |
| Magnesium stearate | 4 | 4 | 4 | 4 | 4 |

### 2. Preparation steps:

1) 10.00 g of PVP K30 was weighed and added into 90.00 g of purified water, and then stirred to dissolve. Thus, 10% of PVPK30 aqueous solution was prepared for later use.
2) Based on the preparation of 100 tablets, the prescribed amounts of API and lactose monohydrate were weighed, and well mixed through an 80 mesh sieve using an incremental analysis.
3) The prescribed amount of sustained-release materials was weighed and mixed with the aforementioned powder through sieving (60-mesh).
4) A suitable amount of adhesives was added to make soft materials, and 20-mesh sieve was used for granulation; the granules were dried at 60 °C for 1 h, and then sorted through a 20-mesh sieve.
5) Magnesium stearate was added in a ratio of 1% by weight of the granules and mixed well.
6) Using a rotary tablet machine, 10 mm of shallow arc circular punches were used for pressing tablets.

### 3. Release detection:

Medium: pH 6.8 phosphate buffer Rolling basket method: 100 RPM

The release rates of 3 tablet samples for each formula were tested, and then the average value was calculated. Samples were taken at 1 h, 2 h, 4 h, 6 h, 8 h, 10 h, 12 h, 16 h, 20 h, and 24 h, and subjected to HPLC analysis to obtain the release curve.

### 4. Results

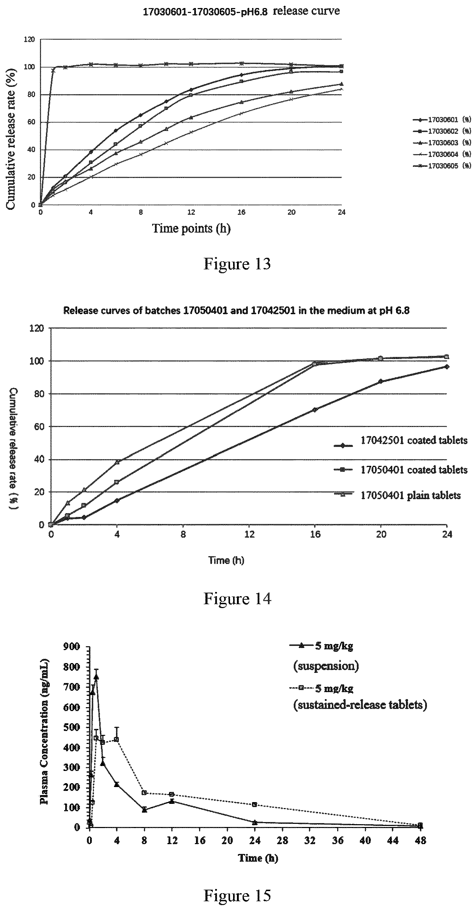

See Table 2 and Figure 13.

**Table 2. The release curves of the samples of quinoline compound sustained-release tablets (specification: 5 mg) for screening of sustained-release materials types and different ratios.**

| Formula | | 1h | 2h | 4h | 6h | 8h | 10h | 12h | 16h | 20h | 24h |
|---|---|---|---|---|---|---|---|---|---|---|---|
| 17030601 | The cumulative release rate (%) | 12.5 | 20.9 | 38.2 | 53.8 | 65.2 | 74.9 | 83.6 | 94.3 | 99.0 | 100.8 |
| | RSD(%) | 16.92 | 18.25 | 18.78 | 19.06 | 15.88 | 11.36 | 8.69 | 5.29 | 1.49 | 0.52 |
| 17030602 | The cumulative release rate (%) | 9.1 | 16.1 | 30.5 | 43.9 | 57.2 | 69.9 | 79.6 | 89.3 | 96.2 | 96.6 |
| | RSD(%) | 10.82 | 4.14 | 1.92 | 3.09 | 4.55 | 6.11 | 6.71 | 3.30 | 1.84 | 0.20 |
| 17030603 | The cumulative release rate (%) | 11.1 | 17.0 | 26.4 | 37.4 | 45.7 | 55.1 | 63.6 | 74.6 | 82.2 | 87.8 |
| | RSD(%) | 14.56 | 6.67 | 1.62 | 0.05 | 2.63 | 209 | 1.31 | 0.14 | 0.52 | 0.31 |
| 17030604 | The cumulative release rate (%) | 6.8 | 11.1 | 20.2 | 29.4 | 36.5 | 44.8 | 52.6 | 66.4 | 76.6 | 84.1 |
| | RSD(%) | 6.30 | 3.11 | 3.51 | 3.34 | 3.16 | 3.46 | 2.74 | 3.91 | 5.26 | 4.58 |
| 17030605 | The cumulative release rate (%) | 97.5 | 99.9 | 102.0 | 101.5 | 101.2 | 102.3 | 102.2 | 102.8 | 101.9 | 100.5 |
| | RSD (%) | 0.16 | 0.48 | 0.22 | 0.22 | 0.49 | 0.30 | 0.08 | 0.39 | 0.15 | 0.51 |

The results indicated that the tablets prepared with sustained-release material HPC HXF had no delayed-release effect in a medium at pH 6.8, with a release rate of over 90% after 1 h. The tablets comprising slow-release materials HPMC K4M at a ratio of 30% and 40% released slowly in the medium at pH 6.8, with a release rate of less than 90% after 24 h. The release rate of the tablets comprising 30% and 40% of sustained-release materials HPMC K750 was moderate, with a release rate of over 95% after 16-20 hours.

### Experimental example 2: The amounts of sustained-release materials in quinoline compound sustained-release tablets

### 1. Formula

See Table 3.

**Table 3. Comparison of different amounts of sustained-release materials used in quinoline compound sustained-release tablets (specification: 5 mg).**

| Components | | | 17050401 (mg/tablet) | 17042501 (mg/tablet) |
|---|---|---|---|---|
| API | | | 5 | 5 |
| HPMC K750 | | | 115 | 160 |
| Lactose monohydrate | | | 255 | 215 |
| 75% ethanol solution | | | 216.9 | 253.4 |
| Magnesium stearate | | | 3.8 | 3.8 |
| Coating solution | Opadry (21K58794) | 5% | Weight gain 3.3% | Weight gain 3.3% |

### 2. Preparation steps:

1) The prescribed amounts of API and lactose were weighed, and well mixed through a 60-mesh sieve using an incremental analysis.
2) The prescribed amount of sustained-release materials was weighed and mixed with the aforementioned powder through sieving for three times.
3) 75% ethanol solution was added to make soft materials, and 20-mesh sieve was used for granulation. The granules were dried at 50 °C for 1 h, and then sorted through a 20-mesh sieve.
4) Magnesium stearate was added in a ratio of 1% by weight of the granules and mixed well.
5) Using a rotary tablet machine, 10 mm of shallow arc circular punches were used for pressing tablets.
6) The coating solution was prepared according to the ratio in the formula, and thus a coating solution at a content of 5% was obtained.
7) A BY300A small coating machine was used for coating, and a certain amount of plain tablets were weighed and placed in a coating pot. The coating was carried out at a weight increase of 3% to prepare the tablets.

### 3. Detection of release curve:

Medium: pH 6.8 phosphate buffer Rolling basket method: 100 RPM

For each plain or coated tablet, the release rates of 3 tablet samples were tested, and then the average value was calculated. Samples were taken at 1 h, 2 h, 4 h, 16 h, 20 h, and 24 h, and subjected to HPLC analysis to obtain the release curve.

### 4. Results

See Table 4 and Figure 14.

**Table 4. Release data of coated and plain tablets for optimizing the amounts of sustained-release materials in quinoline compound sustained-release tablets (specification: 5 mg).**

| Formula | | 1h | 2h | 4h | 16h | 20h | 24h |
|---|---|---|---|---|---|---|---|
| 17042501 coated tablets | The cumulative release rate (%) | 4.0 | 4.4 | 15.0 | 70.3 | 87.4 | 96.5 |
| | RSD(%) | 13.17 | 14.35 | 3.96 | 3.92 | 3.35 | 3.02 |
| 17050401 coated tablets | The cumulative release rate (%) | 5.5 | 11.5 | 26.1 | 97.7 | 101.4 | 102.4 |
| | RSD(%) | 15.39 | 5.96 | 3.27 | 2.56 | 1.11 | 0.78 |
| 17050401 plain tablets | The cumulative release rate (%) | 13.4 | 21.4 | 38.4 | 98.9 | 101.7 | 102.9 |
| | RSD(%) | 20.52 | 19.08 | 12.78 | 0.34 | 1.02 | 0.85 |

According to the data in Table 4 and Figure 14, after reducing the amounts of the sustained-release materials, the release of coated tablets and plain tablets both reached over 95% within 16 hours, approaching complete release. The release of coated tablets was about 10% lower than that of plain tablets in the early stage, but there was almost no difference in release after 16 hours.

### Experimental example 3: Animal experiment

According to the formula of batch 17050401, the amounts were increased to 1000 tablets, and then 18071003 batch samples were produced and subjected to PK testing in beagle dogs. The experimental design was as follows:
The experiment was divided into two stages, and 6 beagles were included, with half male and half female. In the first stage, the experimental animals were orally administered the suspension of active ingredients at a single dose of 5 mg/kg, in which the vehicle was 0.5% sodium carboxymethyl cellulose (0.5% CMC-Na) aqueous solution. After a 7-day washout period, in the second stage, the same experimental animals were orally administered 10 quinoline compound sustained-release tablets (specification 5 mg) once. All animals were fasted overnight before administration and resumed feeding approximately 4 h after administration. 75 mL of KCl-HCl buffer was administered via a gastric tube 15 min before administration. Plasma samples of animals were collected prior to administration as well as at 0.0833 h, 0.25 h, 0.5 h, 1 h, 2 h, 4 h, 8 h, 12 h, 24 h, and 48 h after administration. The concentration of quinoline compounds in plasma samples was determined by the validated LC-MS/MS method. The *in vivo* experimental results in beagle dogs administered with 5 mg/kg of suspension and 5 mg of tablets are shown in Table 5 and Figure 15.

**Table 5. The average pharmacokinetic parameters of quinoline compound suspension or sustained-release tablets in male and female beagle dogs after administration (n = 6).**

| Form of preparation | Suspension | | Sustained-release tablets | |
|---|---|---|---|---|
| Route of administration | PO | | PO | |
| Dosage (mg/kg) | 5.0 | | 5.0 | |

| Pharmacokinetic parameters | Mean | SD | Mean | SD |
|---|---|---|---|---|
| Cₘₐₓ (ng/mL) | 827 | 420 | 620 | 140 |
| Tₘₐₓ (h) | 1.08 | 0.492 | 2.17 | 1.47 |
| T_{1/2} (h) | 8.44 | 3.59 | 8.99 | 2.42 |
| AUC₀₋ₗₐₛₜ (h•ng/mL) | 3520 | 802 | 5842 | 1042 |
| AUC_{0-inf} (h•ng/mL) | 3655 | 884 | 5968 | 1304 |

The experimental results showed that under the conditions of ensuring similar AUC, the Cₘₐₓ of sustained-release tablets was significantly reduced by nearly 25%, that could effectively prevent the incidence of adverse reactions caused by excessive plasma concentrations.

Since HPMC K750, used as a sustained-release material in the tablet formulation, was found to account for a large proportion in the scale-up production, the wear resistance of the tablets during coating was insufficient, the yield was low, and the process compliance decreased. Thus, HPMC K750 was substituted with a mixture of HPMC K4M and K100LV, that not only overcame the problems in the preparation process of tablets, but also obtained the same extended-release effect and pharmacokinetic parameters as those of slow-release tablets in which HPMC K750 was used as sustained-release materials. Therefore, stability studies were carried out on tablet formulations using HPMC K4M and K100LV as sustained-release materials in the subsequent experiment.

### Experimental example 4. Stability study of quinoline compound sustained-release tablets of the present invention

### 1. Formula

See Table 6.

**Table 6. Formulation of quinoline compound sustained-release tablets (specification: 5 mg).**

| Components | | 19082901 (mg/tablet) | |
|---|---|---|---|
| API | | 5 | |
| HPMC K4M | | 57 | |
| HPMC K100LV | | 60 | |
| Lactose monohydrate | | 226.1 | |
| Microcrystalline cellulose PH101 | | 30 | |
| Magnesium stearate | | 1.9 | |
| Coating solution | Opadry (21K58794) | 5% | Weight gain 3% |

### 2. Preparation steps:

1) The prescribed amount of lactose was weighed and passed through a 60-mesh sieve.
2) 1/3 of the prescibed amount of lactose was placed in a wet granulator, stirred for 5 min, and then 1/3 of the pre-determined amount of lactose and 1/2 of the pre-determined amount of quinoline compound in the formula were added and stirred for 5 min. Finally, the remaining amounts of quinoline compounds and lactose were added, and stirred for additional 5 min. Then, the prescribed amounts of HMPC K4M, HPMCK100Lv, and microcrystalline cellulose PH 101 were addeded and stirred for 10 min.
3) Some water was added to make soft materials, the granulation process was performed by passing through a 20-mesh sieve. The materials were dried at 60 °C to the moisture content of < 4%, and then the sorting was carried out with a 20-mesh sieve.
4) Magnesium stearate was added in a ratio of 0.5% by weight of the granules and mixed well.
5) Using a rotary tablet machine, 10 mm of shallow arc circular punches were used for pressing tablets.
6) The coating solution was prepared according to the ratio in the formula, and thus a coating solution at a content of 12% was obtained.
7) A BY300A small coating machine was used for coating, and a certain amount of plain tablets were weighed and placed in a coating pot. The coating was carried out at a weight increase of 3% to prepare the tablets.

3. The stability results are shown in Table 7.

**Table 7. Investigation on the stability of sample (19082901) obtained after optimizing the formula of 5 mg preparation.**

| Accelerated conditions: 40 °C ± 2 °C/75% RH ± 5% RH | | | | | | | |
|---|---|---|---|---|---|---|---|
| Long-term conditions: 25 °C ± 2 °C/60% RH ± 10% RH | | | | | | | |
| **Inspection items** | Time points (h) | | Day 0 | 1 month under accelerated conditions | 2 months under accelerated conditions | 3 months under accelerated conditions | 3 months under long-term conditions |
| | 0 | | 0 | 0 | 0 | 0 | 0 |
| **Release rate (%)** | 1 | | 7.4 | 7.4 | 7.5 | 7.2 | 7.2 |
| | 2 | | 15.2 | 14.8 | 15.0 | 14.8 | 14.8 |
| | 4 | | 31.7 | 30.1 | 30.0 | 30.5 | 30.8 |
| | 6 | | 48.2 | 45.8 | 45.0 | 45.8 | 46.1 |
| | 8 | | 63.8 | 59.8 | 59.8 | 59.9 | 60.0 |
| | 10 | | 76.0 | 71.4 | 70.8 | 71.3 | 71.3 |
| | 12 | | 85.9 | 81.2 | 81.0 | 81.8 | 80.7 |
| | 16 | | 98.4 | 95.7 | 96.6 | 95.3 | 95.2 |
| | 20 | | 103.0 | 100.4 | 102.4 | 101.3 | 102.1 |
| | 24 | | 103.0 | 100.9 | 103.3 | 101.0 | 102.8 |
| **Related substances (%)** | Single foreign material ≤ 0.5% | RRT0.37 | ND | ND | ND | ND | ND |
| | | API-ZA06 (RRT0.94) | 0.06 | 0.06 | 0.06 | 0.06 | 0.06 |
| | | API-ZA05 (RRT1.82) | 0.03 | 0.02 | 0.04 | 0.04 | 0.03 |
| | | IM2 (RRT2.02) | 0.03 | 0.03 | 0.03 | 0.03 | 0.03 |
| | All foreign materials ≤ 2.0% | | 0.12 | 0.11 | 0.13 | 0.13 | 0.11 |

The results indicated that the extended-release effects of the tablets using HPMC K4M and K100LV as sustained-release materials are consistent with those using HPMC K750 as sustained-release materials. In both accelerated and long-term experiments, there was no growth trend in the substances related to the sustained-release tablets of the present invention, and the release curve remained stable and unchanged, demonstrating that the product was stable.

## Claims

1. A quinoline compound sustained-release tablet, **characterized in that** it contains the following raw and auxiliary materials in parts by weight:
1-40 parts of quinoline compounds, 100-300 parts of fillers, 50-200 parts of sustained-release materials and 0.5-4 parts of lubricants;
the filler comprises, but is not limited to, mannitol, microcrystalline cellulose, lactose, starch, corn starch, calcium hydrogen phosphate hydrate, magnesium carbonate, calcium carbonate, purified sucrose and/or glucose;
the sustained-release material comprises, but is not limited to, hydroxypropyl methylcellulose, hydroxypropyl cellulose, povidones, glyceryl behenate, and/or long-chain fatty acids;
the lubricant comprises, but is not limited to, magnesium stearate, calcium stearate, sucrose fatty acid ester, sodium stearyl fumarate, polyethylene glycol, talc, glyceryl behenate and/or stearic acid.

2. The sustained-release tablet according to claim 1, **characterized in that** the filler is lactose and/or microcrystalline cellulose, and preferably, the filler is composed of lactose and microcrystalline cellulose at a mass ratio of 10-70:2-30; the sustained-release material is hydroxypropyl methylcellulose; the lubricant includes but is not limited to magnesium stearate, sodium fumarate, glyceryl behenate, and/or stearic acid.

3. The sustained-release tablet according to claim 1 or 2, **characterized in that** said lactose includes but is not limited to lactose monohydrate, anhydrous lactose, and/or lactose with different particle sizes; said microcrystalline cellulose includes but is not limited to microcrystalline cellulose PH101, PH102, PH301, and/or PH302; said hydroxypropyl methylcellulose includes but is not limited to hydroxypropyl methylcellulose K4M, K100LV, and/or K750; and said lubricant is magnesium stearate.

4. The sustained-release tablet according to claim 1, **characterized in that** it comprises the following raw and auxiliary materials in parts by weight:
5 parts of quinoline compounds, 256 parts of fillers, 117 parts of sustained-release materials and 1.9 parts of lubricants;
the filler is composed of lactose monohydrate and microcrystalline cellulose PH101 at a mass ratio of 10-70:2-30;
said hydroxypropyl methylcellulose is composed of hydroxypropyl methylcellulose K4M and K100LV, and their mass ratio is preferably 57:60;
and said lubricant is magnesium stearate.

5. The sustained-release tablet according to claim 1, **characterized in that** the quinoline compound is a compound as represented by formula I, or an optical isomer thereof, or a solvate thereof, or a pharmaceutically acceptable salt thereof, or a prodrug thereof; 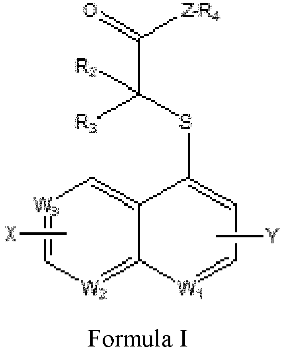 wherein:
Z is selected from O, S or -NH-;
W₁ is selected from N or CR^{a}; W₂ is selected from N or CR^{b}; W₃ is selected from N or CR^{c};
R^{a}, R^{b}, R^{c}, R₂, and R₃ are each independently selected from the group consisting of hydrogen, halogen, cyano, nitro, alkyl, alkenyl, alkynyl, cycloalkyl, heterocyclyl, aryl, heteroaryl, -OR^{d}, -S(O)ₘR^{d}, -C(O)R^{d}, C(O)OR^{d}, -C(O)NR^{e}R^{f}, -NR^{e}R^{f} or NR^{e}C(O)R^{f}, in which said alkyl, alkenyl, alkynyl, cycloalkyl, heterocyclyl, aryl or heteroaryl are each independently and optionally further substituted with one or more of the substituents selected from the group consisting of halogen, cyano, nitro, oxo, alkyl, haloalkyl, hydroxyalkyl, alkenyl, alkynyl, cycloalkyl, heterocyclyl, aryl, heteroaryl, -OR^{d}, -S(O)ₘR^{d}, -C(O)R^{d}, C(O)OR^{d}, -C(O)NR^{e}R^{f}, -NR^{e}R^{f} or NR^{e}C(O)R^{f};
R^{d} is selected from the group consisting of hydrogen, halogen, alkyl, cycloalkyl, heterocyclyl, aryl or heteroaryl, wherein said alkyl, cycloalkyl, heterocyclyl, aryl or heteroaryl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, carboxylic ester group, -C(O)NR^{e}R^{f}, -NR^{e}R^{f} or NR^{e}C(O)R^{f};
R^{e} and R^{f} are each independently selected from the group consisting of hydrogen, alkyl, cycloalkyl, heterocyclyl, aryl or heteroaryl, wherein said alkyl, cycloalkyl, heterocyclyl, aryl or heteroaryl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, and carboxylic ester group; and m is 0, 1, or 2;
X and Y are each independently selected from the group consisting of hydrogen, halogen, cyano, nitro, alkyl, cycloalkyl, haloalkyl and hydroxyalkyl;
Provided that Z is selected from O or S, R₄ is selected from hydrogen, C₁-C₆ alkyl, and cycloalkyl, wherein said alkyl and cycloalkyl are each independently and optionally further substituted with one or more of the substituents selected from the group consisting of halogen, cyano, nitro, hydroxy, oxo, alkyl, haloalkyl, hydroxyalkyl, alkoxy, cycloalkyl, heterocyclyl, aryl, heteroaryl, carboxyl, and carboxylic ester group, -C(O)NR^{e}R^{f}, -NR^{e}R^{f} or NR^{e}C(O)R^{f}; provided that Z is -NH-, R₄ is selected from the group consisting of hydrogen, aryl or heteroaryl, and preferably pyridinyl.

6. The sustained-release tablet according to claim 5, **characterized in that** the quinoline compound is 2-[4-(6-bromoquinolyl)-thio]-2-ethylbutyric acid and/or an optical isomer, a solvate, a pharmaceutically acceptable salt, and a prodrug thereof; the structure of said 2-[4-(6-bromoquinolyl)-thio]-2-ethylbutyric acid is as follows:

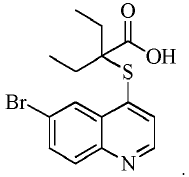

7. The sustained-release tablet according to claim 6, **characterized in that** the quinoline compound is crystalline form I, crystalline form II and/or a sodium salt crystalline form of 2-[4-(6-bromoquinolyl)-thio]-2-ethylbutyric acid.

8. The sustained-release tablet according to claim 7, **characterized in that** there are characteristic peaks where diffraction angles 2θ are 6.5±0.2°, 13.6±0.2°, 14.1±0.2°, 17.7±0.2°, 21.8±0.2°, 22.0±0.2°, 22.8±0.2°, 23.2±0.2°, 24.3±0.2°, 26.8±0.2°, and 27.4±0.2° in the X-ray powder diffraction pattern of the crystalline form I of 2-[4-(6-bromoquinolyl)thio]-2-ethylbutyric acid.

9. The sustained-release tablet according to claim 8, **characterized in that** the relative intensity of the characteristic peak is:
| Diffraction angle 2θ | Relative intensity% | |
|---|---|---|
| 6.5±0.2 | | 36.1 |
| 13.6±0.2 | | 100.0 |
| 14.1±0.2 | | 14.1 |
| 17.7±0.2 | | 13.6 |
| 21.8±0.2 | | 17.0 |
| 22.0±0.2 | | 11.1 |
| 22.8±0.2 | | 16.9 |
| 23.2±0.2 | | 11.1 |
| 24.3±0.2 | | 45.6 |
| 26.8±0.2 | | 15.7 |
| 27.4±0.2 | | 36.5. |

10. The sustained-release tablet according to claim 7, **characterized in that** there are characteristic peaks where diffraction angles 2θ are 7.9±0.2°, 9.5±0.2°, 15.9±0.2°, 18.2±0.2°, 19.1±0.2°, 23.9±0.2°, and 26.1±0.2° in the X-ray powder diffraction pattern of the crystalline form II of 2-[4-(6-bromoquinolyl)thio]-2-ethylbutyric acid.

11. The sustained-release tablet according to claim 10, **characterized in that** the relative intensity of the characteristic peak is:
| Diffraction angle 2θ | Relative intensity% |
|---|---|
| 7.9±0.2100.0 | |
| 9.5±0.27.3 | |
| 15.9±0.228.6 | |
| 18.2±0.2 | 10.0 |
| 19.1±0.26.8 | |
| 23.9±0.222.7 | |
| 26.1±0.26.7 | |

12. The sustained-release tablet according to claim 7, **characterized in that** there are characteristic peaks where diffraction angles 2θ are 6.1±0.2°, 10.5±0.2°, 12.0±0.2°, 14.1±0.2°, 15.9±0.2°, 18.0±0.2°, 21.7±0.2°, 27.6±0.2°, 32.0±0.2°, 33.8±0.2°, and 36.4±0.2° in the X-ray powder diffraction pattern of the sodium salt crystalline form of 2-[4-(6-bromoquinolyl)thio]-2-ethylbutyric acid.

13. The sustained-release tablet according to claim 12, **characterized in that** the relative intensity of the characteristic peak is:
| Diffraction angle 2θ | Relative intensity% | |
|---|---|---|
| 6.1±0.222.2 | | |
| 10.5±0.221.4 | | |
| 12.0±0.230.7 | | |
| 14.1±0.224.3 | | |
| 15.9±0.289.9 | | |
| 18.0±0.238.5 | | |
| 21.7±0.227.9 | | |
| 27.6±0.2 | | 100.0 |
| 32.0±0.2 | | 22.0 |
| 33.8±0.2 | | 34.2 |
| 36.4±0.2 | | 22.4. |

14. A method for preparation of the sustained-release tablet according to claim 7, **characterized in that** the preparation of crystalline form I of 2-[4-(6-bromoquinolyl)-thio]-2-ethylbutyric acid comprises the following steps:
the crude product of 2-[4-(6-bromoquinolyl)-thio]-2-ethylbutyric acid was dissolved in methanol, and then filtered. The filtrate was naturally cooled and allowed to crystallize for 20-24 h. Then, the temperature was controlled to be 15-20 °C, and the filtrate was stirred and allowed to crystallized for 2 h, followed by filtration. The crystals were dried to obtain crystalline form I.

15. A method for preparation of the sustained-release tablet according to claim 7, **characterized in that** the preparation of crystalline form II of 2-[4-(6-bromoquinolyl)-thio]-2-ethylbutyric acid comprises the following steps:
the crude product of 2-[4-(6-bromoquinolyl)-thio]-2-ethylbutyric acid was dissolved in a mixed solution of tetrahydrofuran and dichloromethane (7:4, v/v), and then filtered. The filtrate was evaporated to dry at room temperature to obtain crystalline form II.

16. A method for preparation of the sustained-release tablet according to claim 7, **characterized in that** the preparation of said sodium salt crystalline form of 2-[4-(6-bromoquinolyl)-thio]-2-ethylbutyric acid comprises the following steps:
the crude product of 2-[4-(6-bromoquinolyl)-thio]-2-ethylbutyric acid was dissolved in water, and then the pH value was adjusted to be 12 with NaOH aqueous solution. The resultant solution was stirred for 3 min and filtered. The crystal was dried *in vacuum* to obtain the sodium salt crystalline form.

17. A method for preparation of the quinoline compound sustained-release tablet according to any one of claims 1-8, **characterized in that** it is prepared using wet granulation, dry granulation, direct powder compression or membrane controlled-release techniques, and preferably wet granulation.

18. The preparation method according to claim 17, **characterized in that** the wet granulation comprises the following steps:
a) The raw and adjuvant materials are weighed according to the ratio in the formula, and lactose is passed through a 60-mesh sieve;
b. 1/4-1/3 of the pre-determined amount of lactose in the formula is placed in a wet granulator, stirred for 5-10 min, and then 1/4-1/3 of the pre-determined amount of lactose and 1/3-1/2 of the pre-determined amount of quinoline compound are added and stirred for 5-10 min. Finally, the remaining amounts of lactose and quinoline compounds are added, and stirred for 5-10 min. Then, microcrystalline cellulose and hydroxypropyl methylcellulose are added and stirred for 10-20 min;
c. Water is added to make soft materials and granules, and then wet granules are dried to a moisture content of 1.0%~4.0%, followed by sorting;
d. Magnesium stearate is mixed with the granules prepared in step c, and then the mixture is pressed and coated to obtain the tablets.

19. The preparation method according to claim 18, **characterized in that** in step b, 1/3 of the pre-determined amount of lactose in the formula is placed in a wet granulator, stirred for 5 min, and then 1/3 of the pre-determined amount of lactose and 1/2 of the pre-determined amount of quinoline compound are added and stirred for 5 min. Finally, the remaining amounts of lactose and quinoline compounds are added, and stirred for 5 min. Then, microcrystalline cellulose and hydroxypropyl methylcellulose are added and stirred for 10 min.

20. The preparation method according to claim 18, **characterized in that** in step c, the granulation process involves passing through a 20-mesh sieve, the drying is carried out at 60 °C, and the sorting is performed with a 20-mesh sieve; and/or, in step d, the tablet is coated at a weight gain of 2-4%.

21. A crystalline form of 2-[4-(6-bromoquinolyl)-thio]-2-ethylbutyric acid, **characterized in that** it is crystalline form I of 2-[4-(6-bromoquinolyl)-thio]-2-ethylbutyric acid, and there are characteristic peaks where diffraction angles 2θ are 6.5±0.2°, 13.6±0.2°, 14.1±0.2°, 17.7±0.2°, 21.8±0.2°, 22.0±0.2°, 22.8±0.2°, 23.2±0.2°, 24.3±0.2°, 26.8±0.2°, and 27.4±0.2° in the X-ray powder diffraction pattern thereof.

22. The crystalline form according to claim 21, **characterized in that** the relative intensity of the characteristic peaks is:
| Diffraction angle 2θ | Relative intensity% | |
|---|---|---|
| 6.5±0.2 | | 36.1 |
| 13.6±0.2 | | 100.0 |
| 14.1±0.2 | | 14.1 |
| 17.7±0.2 | | 13.6 |
| 21.8±0.2 | | 17.0 |
| 22.0±0.2 | | 11.1 |
| 22.8±0.2 | | 16.9 |
| 23.2±0.2 | | 11.1 |
| 24.3±0.2 | | 45.6 |
| 26.8±0.2 | | 15.7 |
| 27.4±0.2 | | 36.5. |

23. A crystalline form of 2-[4-(6-bromoquinolyl)-thio]-2-ethylbutyric acid, **characterized in that** it is crystalline form II of 2-[4-(6-bromoquinolyl)-thio]-2-ethylbutyric acid, and there are characteristic peaks where diffraction angles 2θ are 7.9±0.2°, 9.5±0.2°, 15.9±0.2°, 18.2±0.2°, 19.1±0.2°, 23.9±0.2°, and 26.1±0.2° in the X-ray powder diffraction pattern thereof

24. The crystalline form according to claim 23, **characterized in that** the relative intensity of the characteristic peaks is:
| Diffraction angle 2θ | Relative intensity% |
|---|---|
| 7.9±0.2100.0 | |
| 9.5±0.27.3 | |
| 15.9±0.228.6 | |
| 18.2±0.2 | 10.0 |
| 19.1±0.26.8 | |
| 23.9±0.222.7 | |
| 26.1±0.26.7. | |

25. A crystalline form of 2-[4-(6-bromoquinolyl)-thio]-2-ethylbutyric acid, **characterized in that** it is the sodium salt crystalline form of 2-[4-(6-bromoquinolyl)-thio]-2-ethylbutyric acid, and there are characteristic peaks where diffraction angles are 6.1±0.2°, 10.5±0.2°, 12.0±0.2°, 14.1±0.2°, 15.9±0.2°, 18.0±0.2°, 21.7±0.2°, 27.6±0.2°, 32.0±0.2°, 33.8±0.2°, and 36.4±0.2° in the X-ray powder diffraction pattern thereof.

26. The crystalline form according to claim 25, **characterized in that** the relative intensity of the characteristic peaks is:
| Diffraction angle 2θ | Relative intensity% |
|---|---|
| 6.1±0.222.2 | |
| 10.5±0.221.4 | |
| 12.0±0.230.7 | |
| 14.1±0.224.3 | |
| 15.9±0.289.9 | |
| 18.0±0.238.5 | |
| 21.7±0.227.9 | |
| 27.6±0.2 | 100.0 |
| 32.0±0.2 | 22.0 |
| 33.8±0.2 | 34.2 |
| 36.4±0.2 | 22.4. |
